# EUROPÄISCHE PATENTSCHRIFT

(11) **EP 0 714 243 B1**
(45) Veröffentlichungstag und Bekanntmachung des Hinweises auf die Patenterteilung: **28.10.1998**
(21) Anmeldenummer: 94924699.5
(22) Anmeldetag: 17.08.1994
(51) Int. Cl.: A24F 47/00

(54) **RAUCH- ODER INHALATIONSVORRICHTUNG**
SMOKING OR INHALATION DEVICE
DISPOSITIF POUR FUMER OU INHALER

(30) Priorität: 19.08.1993 DE 4328243
(43) Veröffentlichungstag der Anmeldung: 05.06.1996
(73) Patentinhaber: Mielordt, Sven, D-10963 Berlin (DE)
(72) Erfinder: Mielordt, Sven, D-10963 Berlin (DE)
(74) Vertreter: Christiansen, Henning, Dipl.-Ing.
(86) Internationale Anmeldenummer: DE9400991
(87) Internationale Veröffentlichungsnummer: WO9505094

(56) Entgegenhaltungen:
- EP-A- 0 358 002
- DE-U- 9 218 005
- GB-A- 526 678
- US-A- 2 104 266
- US-A- 4 141 369
- US-A- 5 095 921

## Beschreibung

Die Erfindung betrifft eine Vorrichtung zum Rauchen von Tabak oder eines anderen Rauchmittels oder zum Inhalieren von Aerosolen, welche bei Erwärmung einer aerosolbildenden Substanz gebildet werden.

Das Rauchen von vorzugsweise Tabakwaren ist eine weitverbreitete Anwendungsform von Genußmitteln. Die bei der Verbrennung des Tabaks entstehenden typischen Begleitprodukte sind jedoch in hohem Maße gesundheitsschädigend, wenn sie z.B. mittels des Tabakrauchs über das Atmungssystem in den menschlichen Körper gelangen. Das Vergiftungspotential rauchbarer Genußmittel, das bei Verbrennungstemperaturen von 800 bis 1000°C entsteht, besteht neben der im Verhältnis meist geringeren toxischen Wirkung des eigentlichen Genußmittels, aus einer Menge Teer, Kondensat bzw. schwerer flüchtigen Cancerogenen sowie Kohlenmonoxid und anderen toxischen anorganischen Stoffen, beispielsweise Schwermetallen. Versuche haben ergeben, daß die Freisetzung der für einen Rauchgenuß wichtigen Alkaloide und Wirkstoffe, beispielsweise des Nikotins, bei weitaus niedrigeren Temperaturen erfolgt: sogar schon ab ca. 100 Grad Celsius.

Entsprechende Geräte können aber auch im Dienste der Gesundheit (beispielsweise zur Raucherentwöhnung) verwendet werden, wenn durch den Erhitzungsvorgang pharmazeutisch wirksame Aerosole aus Substanzen in dem sie passierenden Luftstrom überführt werden.

Aus der EP-A2 358 002 bzw. der DE-U 92 18 005.1 ist ein derartiges Rauchgerät bekannt, bei dem die Erwärmung des Rauchmittels durch ein Strömungsmedium, vorzugsweise Luft, erfolgt, welche zuvor durch elektrische Energie erhitzt worden ist. Für das Erhitzen der Luft ist ein batteriegespeistes Widerstandsheizelement vorgesehen. Die konvektive Erwärmung des Rauchmittels erfolgt auf eine Temperatur unterhalb der Verbrennungstemperatur, wodurch die Entstehung von Schadstoffen verhindert bzw. vermindert und die Entwicklung von Aerosolen bzw. von den Rauchgenuß stimulierenden Stoffen gefördert werden soll.

Bei der bekannten Vorrichtung wird das zu einer zylindrischen Form verpreßte Rauchmittel in seiner gesamten Länge von der erwärmten Luft stets im gleichen Richtungssinn durchströmt. Weiterhin kann während der gesamten Rauchzeit die Position des im wesentlichen kompakt oder erheblich verdichtet angeordneten Rauchmittels bezüglich des warmen Strömungsmittels bzw. der elektrischen Wärmequelle nicht verändert werden.

Daraus resultiert der Nachteil, daß nur die unmittelbar von der zuvor erhitzten Luft beaufschlagten Rauchmittelanteile in Oberflächenbereichen auf die gewünschte Temperatur erwärmt und zur Abgabe der den Rauchgenuß bestimmenden Aerosole angeregt werden können. Bei längerem Einwirken der erhitzten Luft auf diese Rauchmittelteilmenge können örtliche Überhitzungen eintreten, wodurch die Entzündung oder zumindest Verkohlung und damit eine Erzeugung von Schadstoffen nicht auszuschließen ist. Desweiteren wird die vorgesehene Rauchmittelmenge in nachteiliger Weise nur teilweise genutzt, da deren größter Teil nicht mehr mit einer ausreichenden Temperatur erwärmt wird. Das führt nicht nur zu einem relativ hohen Energiebedarf, da die Heizintervalle unabhängig von der Rauchintensität festgelegt sind, sondern auch zu einer schlechten Ausnutzung der Substanz.

Ausgehend von den Mängeln des Standes der Technik liegt der Erfindung die Aufgabe zugrunde, eine Vorrichtung der eingangs genannten Gattung zu schaffen, welche mit einfachen Mitteln eine möglichst weitgehende Ausnutzung der vorhandenen Rauchmittelmenge, insbesondere auch zur Erzeugung eines gewünschten Rauchprofils, ermöglicht.

Diese Aufgabe wird mit den Merkmalen der Ansprüche 1 bzw. 2 gelöst.

Die Erfindung schließt die Erkenntnis ein, daß eine gleichmäßige und effektive Ausnutzung der Substanz, der die Aerosole zu entziehen sind, dann erreichbar ist, wenn das Rauchmittel insbesondere (sequenziell) gleichmäßig auf eine Temperatur unterhalb der Gluttemperatur erhitzt wird, bei der die Aerosolbildung ohne Entstehen von gefährlich viel gesundheitsschädlichen Zusatzprodukten erfolgt. Diese Bedingung läßt sich insbesondere dann erfüllen, wenn die relativ fein verteilte Rauchmittelmenge - gegebenenfalls in Teilmengen - konvektiv durch Anströmung mittels eines Trägergases, insbesondere erhitzter Luft, derart erwärmt wird, daß die Volumenkörper der Partikel des Rauchmittels in Oberflächennähe durch das herangeführte Trägergas ausschließlich unmittelbar beaufschlagt und möglichst vollständig durchwärmt werden. Damit wird vermieden, daß - wie beispielsweise bei patronenartigen Anordnungen - das Trägergas in bereits abgekühltem Zustand noch in tiefergelegene Bereiche des Rauchmittels eindringt, die dann nur unzureichend erwärmt werden, was zu einer Ineffektivität hinsichtlich der Ausnutzung der zur Verfügung stehenden Aerosole führen wurde. Hierbei konnten auch die bekannten auf Verbrennung beruhenden Rauchgeräte keine baulichen Vorbilder liefern.

Würde man, wie bei eingangs beschriebener strömungstechnisch fixierter Patronenanordnung, die Luft aber andererseits auf eine solche Temperatur erwärmen, daß auch weiter innen liegende Volumenbereiche des Rauchmittels ausreichend erwärmt werden, wäre eine Überhitzung - und damit Entzündung der zuerst angeströmten Volumenbereiche die Folge. Auch würden damit vermehrt gesundheitsschädliche Substanzen frei werden.

Die gewünschten Temperaturverhältnisse sind gewährleistet, wenn die temperaturmäßig beaufschlagte Oberfläche einer Rauchmittelmenge groß im Verhältnis zu dem von der Oberfläche eingeschlossenen Rauchmittelvolumen gewählt wird. Ein gewünschtes Rauchprofil ist dann zum Beispiel softwaremäßig durch benutzerdefinierte Rauchmitteltemperaturen, entsprechenden Rauchmittelvorschub sowie eine Oszillation der Relativpositionierung von Rauchmittel und Heißluftstrahl bestimmbar.

Durch die derart bemessene Temperatur wird die Entstehung organischer Cancerogene und die Freisetzung von anorganischen Giftstoffen (Schwermetalle und Kohlenmonoxid) in günstiger Weise unterbunden oder zumindest erheblich vermindert.

Erfolgt die Erwärmung entsprechend einer vorteilhaften Weiterbildung der Erfindung impulsweise, so kann die Temperatur der Luft auch über der Entzündungstemperatur des Rauchmittels liegen, wenn dafür gesorgt ist, daß dessen Temperatur aufgrund der zusammenwirkenden thermischen Zeitkonstanten des Rauchmittels und seiner Umgebung nicht bis zur Entzündungstemperatur ansteigt. Auf diese Weise kann die Temperatur der maximalen Ausnutzung des Rauchmittels bereits jeweils sehr schnell erreicht werden, so daß der Luftanteil mit nur kleinem Anteil von Aerosolen in der Anfangsphase des Erwärmungsvorgangs klein gehalten ist und damit für den Benutzer nicht unangenehm in Erscheinung tritt.

Die Gluttemperatur des Rauchmittels bildet ein gutes Erkennungskriterium für den Prozeß, da mit der damit stark ansteigenden Rot- bzw. Infrarotstrahlung ein gutes Regel- bzw. Ausschlußkriterium für die Wärmezufuhr gegeben ist. Diese Glut tritt auch bei Verwendung von Inertgas als Wärmetransportmedium auf. Durch Vermeidung der Glut bleibt der Vorgang auch bei Benutzung von Luft als Trägergas gut kontrollierbar, da eine unerwünschte Wärmezufuhr durch den sonst einsetzenden Verbrennungsvorgang unterbleibt.

Entsprechend einer bevorzugten Ausführungsform der Erfindung ist die gesamte Rauchmittelmenge in einem Vorratsbehälter angeordnet, der eine Mehrzahl, im wesentlichen gleichgroßer Kammern aufweist. Diese Kammern sind jeweils mit einer entsprechenden Teilmenge des Rauchmittels gefüllt. Die Erwärmung der Rauchmittelteilmengen erfolgt konvektiv durch einen Luftstrom, welcher zuvor in einem Heißluftgenerator mittels einer vorzugsweise elektrischen Heizvorrichtung auf die erforderliche Temperatur erhitzt worden ist. Die zu erwärmende Luftmenge wird aus der Umgebung der Rauchvorrichtung dem Heißluftgenerator durch die Saug- bzw. Inhalationstätigkeit des Rauchers zugeführt. Um die gesamte Rauchmittelmenge sequenziell im wesentlichen gleichmäßig oder gleichartig zu erwärmen und die gesamte nutzbare Aerosolmenge aus dem Rauchmittel zu entwickeln, erfolgt eine partielle Erwärmung der Teilmengen des Rauchmittels. Dazu ist eine Relativbewegung zwischen dem Heißluftstrom und den zu erwärmenden Rauchmittelteilmengen vorgesehen. Besonders einfach ist dabei eine Bewegung des Rauchmittelbehälters bei feststehendem Heißluftstrom konstruktiv zu realisieren. Bei einem zylindrischen Vorratsbehälter geringer Höhe erfolgt durch vorgesehene Antriebsmittel eine stetige Drehbewegung oder eine schrittweise Drehung, wobei in günstiger Weise die Rauchmittelmenge kreisegmentweise unter dem Heißluftstrom geführt wird. Der Vorratsbehälter ist dementsprechend kreisegmentweise in gleichgroße Kammern unterteilt, in denen jeweils die gleiche Menge des zur Verfügung stehenden Rauchmittels vorrätig gehalten ist. Für die Erzeugung individueller Rauchprofile ist es günstig, die Bewegung des Rauchmittels in Abhängigkeit von der Menge und Temperatur der zugeführten Luft zu steuern.

Zur Stabilisierung der Relativpositionierung der Rauchmittelteilmengen ist ein strömungsdurchlässiges Rückhaltegitter ratsam.

Möglich ist auch die Verwendung von Fertigtablinen oder sonstigen Flachformlingen, die in das Gerät eingesetzt werden und das Rauchmittel anströmbar enthalten.

Entsprechend einer günstigen Weiterbildung der Erfindung ist der Vorratsbehälter als flacher Quader ausgebildet und weist eine quaderförmige Kammerung auf. Um eine gleichmäßige Erwärmung der in den jeweiligen Kammern befindlichen Rauchmittelmenge zu gewährleisten, ist ein Antrieb vorgesehen, der den Vorratsbehälter translatorisch bewegt. Die Bewegung unter dem Heißluftstrom erfolgt stetig oder schrittweise nach einem entsprechenden vorgebbaren Programm.

Die Erfindung weist - mindestens in einigen ihrer Aspekte - noch die folgenden Vorteile oder Weiterbildungen auf:

Um eine konstante Erwärmung unabhängig von der Saugleistung (bzw. Inhalationsmenge) des jeweiligen Rauchers zu gewährleisten, ist eine strömungsdurchsatzabhängige Regelung der dem Heißluftgenerator zugeführten Elektroenergie vorgesehen. Unter Ausnutzung der physikalischen Zusammenhänge zwischen Strömungsgeschwindigkeit, Strömungsmittelmenge und Druck in einer Strömung wird die Menge der durch den Raucher durch Ansaugen bzw. Inhalieren in den Heißluftgenerator geförderten Umgebungsluft mittels eines Differenzdruck-Sensors erfaßt und einem Regler zugeführt, der zur entsprechenden Änderung der Heizleistung vorgesehen ist. Bei kleiner Wärmekapazität des Heizelements/Heizmittels kann dieses dem gewünschten Temperaturprofil mit kleiner Zeitkonstante folgen.

Der Heißluftgenerator weist zur Lufterhitzung eine elektrische Heizvorrichtung auf, durch die die angesaugte Umgebungsluft auf einen Temperaturwert angehoben wird, welcher oberhalb der für eine pyrolytische Aerosolbildung aus dem Rauchmittel erforderlichen Temperatur liegt. Diese Überhitzung ist zum Ausgleich der Zeitkonstante erforderlich, die sich aus dem Wärmeverlust längs der konstruktionsbedingten Wegstrecke zwischen Erhitzungsort innerhalb des Heißluftgenerators und Ort der Erwärmung der Rauchmittelmenge zwangsläufig ergibt. Entsprechend einer anderen vorteilhaften Weiterbildung der Erfindung ist die Heizvorrichtung als streifenförmiges Folien-Element ausgebildet. Um einerseits den Strömungswiderstand zu verringern und andererseits den Flächenkontakt des Folien-Elements mit dem zu erhitzenden Strömungsmedium zu erhöhen, ist das Folien-Element U-förmig gebogen und weist auf seiner Fläche eine Mehrzahl von Durchbrüchen auf. Es erstreckt sich in Strömungsrichtung der zu erhitzenden Luft, wobei die Luft in die U-förmige Öffnung strömt. Die Durchbrüche sind kreis- und/oder schlitzförmig ausgebildet. Nach einer zusätzlichen Ausführungsform der Erfindung besteht die Heizvorrichtung aus einem bandförmigen Grundkörper mit geringer Bauchigkeit, an dessen Seitenwänden oberflächenvergrößernde Erhebungen in Form von runden Noppen oder kegligen Zapfen vorgesehen sind.

Entsprechend einer anderen günstigen Weiterbildung der Erfindung wird die Gefahr der Schadstoffentwicklung zusätzlich verrringert, indem an Stelle von Luft für die konvektive Erwärmung des Rauchmittels ein inertes Gas verwendet wird.

Zur optimalen Ausnutzung des Rauchmittels ist eine nahezu isotherme Erwärmung der aktivierten Rauchmittelteilmengen wünschenswert. Dem steht jedoch zu einem gewissen Grade immer die physikalische Gesetzmäßigkeit entgegen, daß bei Anströmung mit einem Trägergas vorrangig die oberflächennahen Bereiche der Rauchmittelpartikel erfaßt werden.

Gegenüber dem bisherigen Stand der Technik weist die vorliegende Erfindung alleine schon durch die Anströmung des Rauchmittels relativ großflächig und mit geringer Schichtdicke in Richtung zum Strahl des erhitzten Trägergases eine sehr gleichmäßige Erwärmung der (sequenziell) aktivierten Partikel mit im Verhältnis zur Partikelgröße hohen Eindringtiefe auf, was im Text dieser Schrift durch die Formulierung "sequenziell gleichartig" bzw. "gleichmäßig" verdeutlicht werden soll.

Durch Bündelung von Energiestrahlung in den Volumenbereich der aktivierten Rauchmittelzone kann ebenfalls die Aerosolfreisetzung angeregt werden. Verwendet man Licht- oder kurzwellige Infrarotstrahlung, so werden, wie bei Anströmung mit erhitztem Trägergas, die Oberfächen der Partikel etwas heißer als deren Kerne, weil die Strahlung an der Oberfläche der Partikel absorbiert wird.

Verwendet man jedoch längerwellige Strahlung bis in den Mikrowellen- oder Radiofrequenzbereich, wo wird die Strahlungsenergie vom Rauchmittel volumenmäßig absorbiert, unter anderem auf Grund dielektrisch verlustbehaftetem (Absorptions-)Verhalten im Hochfrequenzfeld. So läßt sich erreichen, daß die Kerne der Rauchmittelpartikel tendenziell wärmer werden als deren Oberflächen, weil das immer zur Aerosolabführung erforderliche Trägergas dann konvektiv tendenziell kühlend auf die Oberflächen wirkt.

Strömt man gleichzeitig mit erhitztem Trägergas an und sendet penetrierend absorbierbare Energiestrahlung in die aktive Rauchmittelzone, so kann bei geeigneter Aufteilung der Heizleistung auf das Trägergas und die Strahlungsenergie auch bei endlicher Eindringtiefe in die Partikel eine nahezu gradientenfreie Erwärmung der Partikel geschehen; bzw. sogar ein solcher Temperaturgradient erzeugt werden, daß der Kern der Partikel heißer wird als deren angeströmte Oberfläche, um durch Erhöhung des Dampfdrucks der Aerosole im Kern der Partikel die Diffusion zur Oberfläche und damit den Wirkungsgrad im Verhältnis zur Toxizität zu perfektionieren.

Eine stoßweise Kernüberhitzung mittels Strahlung kann über Lockerung der Partikelstruktur und Vergrößerung der aktiven Oberfläche ebenfalls hilfreich sein. Die Bündelung der Strahlung erfolgt normalerweise ringförmig oder von mindestens zwei gegeneinander geneigten Flächen. Der Rauchmittelteller kann als Strahlenreflektor ausgebildet werden.

Im Rahmen von (softwarekontrollierten) Rauchprofilen ist es sinnvoll, das Rauchmittel in mehreren Recylingdurchgängen "abzufahren". Hierfür wird ein Vorschub eingestellt, der die aktiven Rauchmittelvolumenbereiche annähernd proportional zu durchgesaugter Trägergasmenge und Solltemperatur verschiebt, wobei die Anfangsphase eines Zuges wegen der nötigen Aufwärmung womöglich einen geringeren oder gar keinen Vorschub auslöst. Sobald nach einem Durchgang die gesamte nutzbare Rauchmittelmenge flächenmäßig überstrichen wurde, wird üblicherweise im nächsten Durchgang die Temperatur erhöht, gleichzeitig wegen der bereits eingetretenen Auslaugung der Vorschub überproportional erhöht. Spätestenes nach einem dritten Durchgang mit progressiv erhöhten Parametern empfindet der Benutzer das Rauchmittel normalerweise als vollständig ausgelaugt und nicht mehr weiter genießbar. Günstig ist eine gewisse überlagerte Oszillation der Relativposition Trägergasstrahl-aktive Zone insbesondere bei hohen Temperaturen.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
Figur 1 die schematisierte Darstellung eines Längsschnittes durch eine bevorzugte Ausführungsform der Erfindung (entlang der Linie A-A in Figur 2),
Figur 2 eine weitere Schnittdarstellung des Ausführungsbeispiels gemäß Figur 1,
Figuren 3 bis 3f andere vorteilhafte Weiterbildungen der in den Figuren 1 und 2 gezeigten Erfindung,
Figur 4 eine weitere vorteilhafte Ausführungsform der Erfindung in vereinfachter Darstellung
Figur 5 eine schematisierte Darstellung einer vorteilhaften Varianten der Ausführungsform gemäß Figur 4,
Figur 6 eine weitere günstige Ausführungsform der Erfindung in einer Schnittdarstellung sowie
Figur 7 eine andere vorteilhafte Ausführungsform der Erfindung im Schnitt.

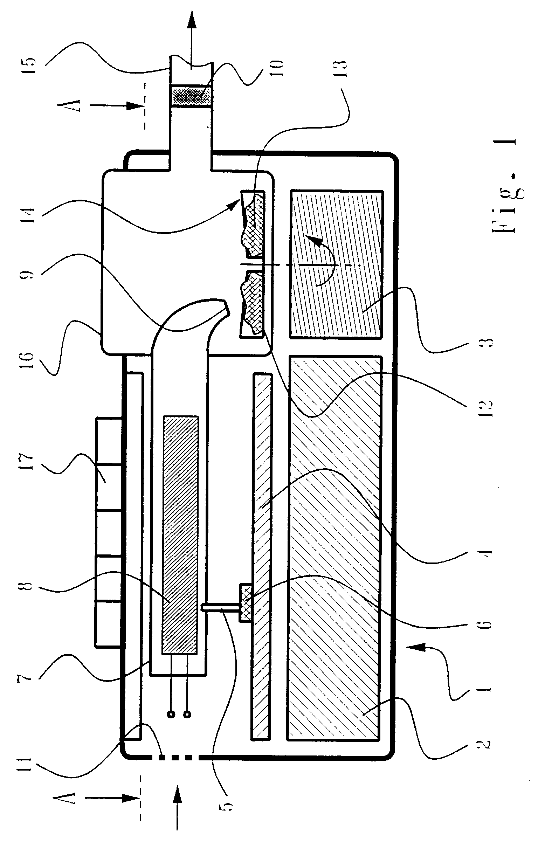

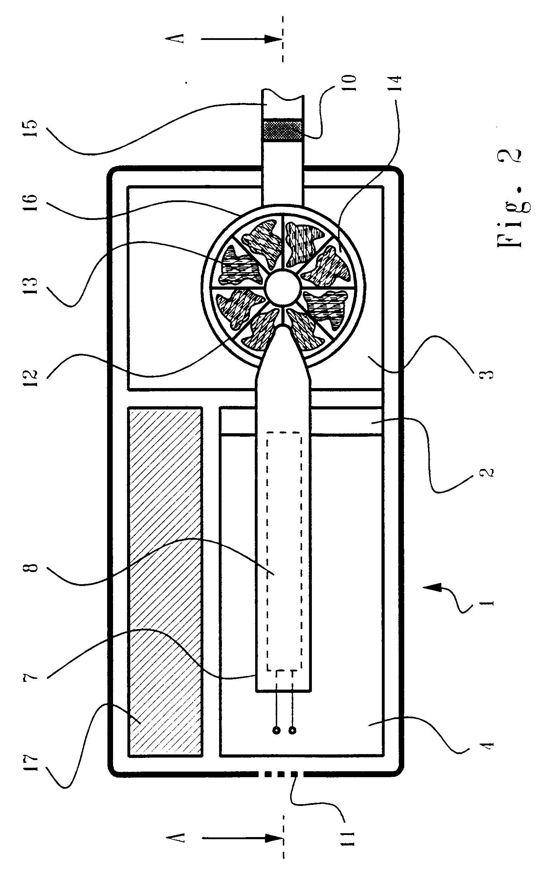

Die in Figur 1 als Längsschnitt längs der Linie A...A gemäß Figur 2 dargestellte Vorrichtung 1 zum Rauchen von Tabak oder anderen Rauchmitteln weist als wesentliches Bauteil einen Heißluftgenerator 7 auf. Die aus dem Heißluftgenerator 7 austretende erhitzte Luft wird zur konvektiven Erwärmung des Rauchmittels 13 benutzt. Der Raucher saugt während des Rauchvorgangs über einen Stutzen 15 die erforderliche Luftmenge durch einen in der Gehäusewandung vorgesehenen Lufteintrittsöffnung 11. In diesen Strömungsweg ist der Heißluftgenerator 7 derart eingebunden, daß die angesaugte Umgebungsluft von der Lufteintrittsöffnung 11 direkt in den Heißluftgenerator 7 gelangt und an einer Heizvorrichtung 8 vorbeiströmen kann. Dadurch entsteht eine Erwärmung des Rauchmittels in dessen oberflächennahen Bereichen.

Die Luftaustrittsdüse 9 des Heißluftgenerators 7 ragt in die Rauchkammer 16 der Rauchvorrichtung 1 und ist mit ihrer Austrittsöffnung dicht oberhalb des in einem Vorratsbehälter 12 eingebrachten Rauchmittels 13 angeordnet. Die erhitzte Luft weist an dieser Stelle eine Temperatur auf, bei welcher das Rauchmittel 13 pyrolytisch unter Abgabe von Aerosolen umgewandelt wird. Durch die konvektive Erwärmung des Rauchmittels 13 ist eine Verbrennung, bei der bei Temperaturen von etwa 800°C gesundheitsschädliche Produkte entstehen, unterbunden. Für die Bildung von Aerosolen, die für den eigentlichen Rauchgenuß wesentlich sind, ist es günstig, wenn das betroffene Partikelvolumen sequenziell im wesentlichen gleichmäßig bzw. gleichartig auf die jeweilige Pyrolyse-Temperatur erwärmt wird. Deshalb ist die in der Rauchkammer 16 in dem Vorratsbehälter 12 befindliche Rauchmittelmenge in mehrere Teilmengen aufgeteilt und in den einzelnen Kammern 14 des Vorratsbehälters 12 untergebracht.

Die Antriebsvorrichtung 3 versetzt den Vorratsbehälter 12 in eine Drehbewegung, so daß die einzelnen Teilmengen des Rauchmittels an dem Heißluftstrom am Ausgang der Düse 9 vorbeigeführt werden. Unabhängig davon, ob die Rotation des Vorratsbehälters 12 zur Relativbewegung zwischen Heißluftstrom und Rauchmittel kontinierlich oder schrittweise entsprechend einer wählbaren Steuerung erfolgt, werden die einzelnen Teilmengen des Rauchmittels gleichartig erwärmt und mit hohem Ausnutzungsgrad zur Aerosolabgabe herangezogen. Dies gewährleistet in günstiger Weise einen gleichmäßigen, hohen Rauchgenuß und eine verlustarme Verwertung der gesamten Rauchmittelmenge. Für die vollständige Ausnutzung der zum Genuß vorgesehenen Rauchmittelmenge ist eine gleichmäßige und von der Intensität des Rauchens (starkes Saugen oder geringes Inhalieren) unabhänge Temperatur des Heißluftstromes wesentlich. Dafür ist eine (nicht näher dargestellte) Regelungseinrichtung vorgesehen, welche über einen Drucksensor 6 die Strömungsgeschwindigkeit innerhalb des Heißluftgenerators 7 ermittelt und danach die Energiezufuhr für die, vorzugsweise elektrische, Heizvorrichtung 8 regelt. Die strömungstechnische Anbindung des Drucksensors 6 erfolgt über eine Rohrleitung 5.

Für den Betrieb der Rauchvorrichtung 1 ist eine Batterie 2 vorgesehen, die die Energieversorgung für den Motorantrieb 3 und eine Elektronik-Baugruppe 4 gewährleistet. Die Bedien-Einheit 17 ermöglicht über entsprechende Tasten ein bequemes Ein- und Ausschalten der Vorrichtung 1, das Einstellen des erforderlichen Bereichs der Heißluft-Temperatur bzw. die Wahl der Drehzahl des Motors 3. Dadurch ist es auf einfache Weise möglich, ein bestimmtes Rauchprofil - gegebenenfalls softwaremäßig - vorzugeben. Das im Saugstutzen 15 vorgesehene Filter 10 hält mögliche unerwünschte Partikel, die durch den Heißluftstrom innerhalb der Rauchkammer 16 bewegt werden, zurück.

Figur 2 zeigt die Schnittansicht längs der Linie A...A gemäß Figur 1 in schematisierter Darstellung. Der Vorratsbehälter 12 ist als flacher, einseitig geöffneter Kreiszylinder ausgebildet. Er weist eine Kammerung in mehrere, gleichgroße Kreissegmente 14 auf, in welche jeweils eine im wesentlichen gleichgroße Rauchmittelmenge 13 eingebracht worden ist.

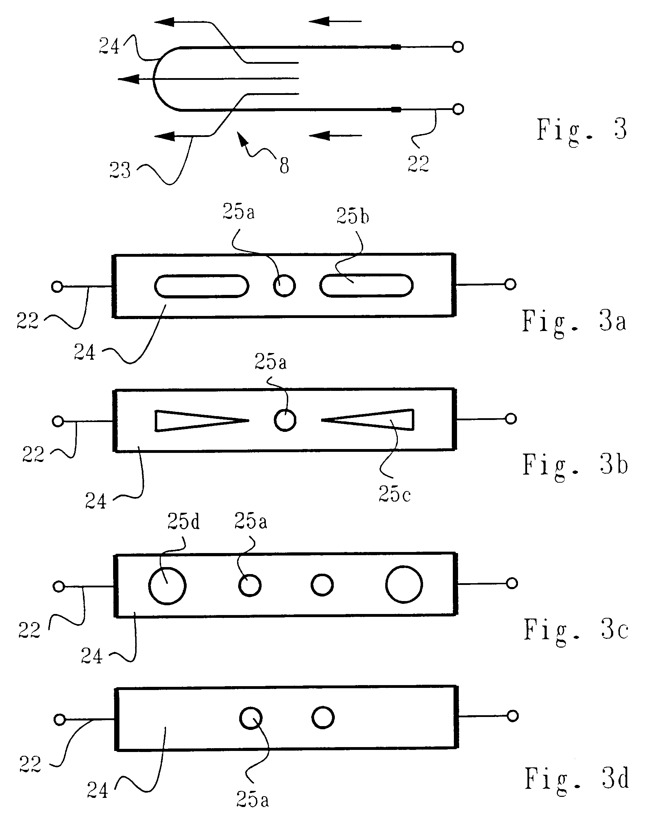

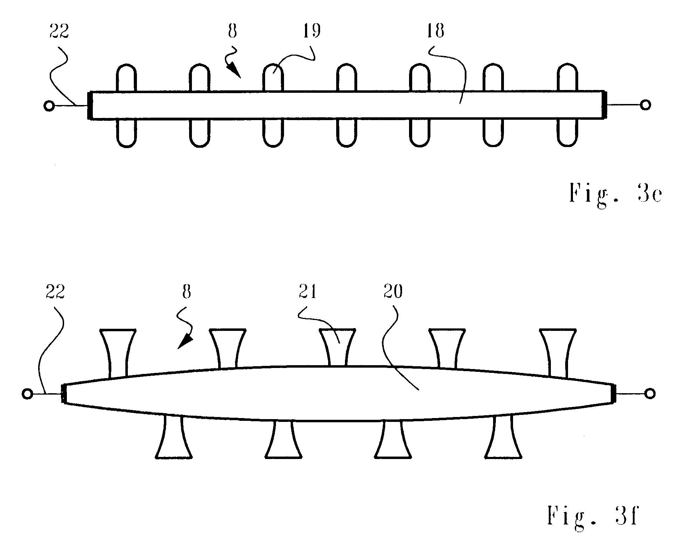

In den Figuren 3, 3a bis 3f sind als Weiterbildung der Erfindung vorteilhafte Ausführungen der in den Figuren 1 und 2 vorgesehenen Heizvorrichtung 8 in schematisierter Form dargestellt. Die Heizvorrichtung 8 ist als Folien-Heizelement ausgebildet (Figur 3). Die Heizfolie 24 ist U-förmig gebogen und wird zwischen den Schenkeln des U angeströmt. Die Luftströmung 23 (angedeutet durch Pfeile) durchdringt die Heizfolie 24 über verschiedene Durchbrüche 25a bis 25 d, die als kreisförmige Öffnungen oder geometrisch gestaltete Schlitzen ausgebildet und kombiniert sind (Figuren 3a bis 3d). Die Heizfolie weist in vorteilhafter Weise eine geringe Wärmekapazität auf, wodurch günstige Bedingungen für ein Leerrauchen und die Erzeugung bestimmter Rauchprofile gegeben sind.

Wird die elektrische Heizvorrichtung 8 als kompaktes quaderförmiges, insbesondere als dünnwandiger Hohlkörper ausgestaltetes, Heizelement 18, 20 mit geradliniger oder bau chiger Wandung ausgebildet, sind oberflächenvergrößernde Noppen 19 oder Zapfen 21 vorteilhaft, um den Wärmeübergang zur vorbeiströmenden Luft zu vergrößern und gleichzeitig auf vorteilhafte Weise eine mechanisch günstige Abstützung des Heizelements innerhalb des Strömungskanals zu gewährleisten (Figuren 3e und 3f).

Zum Anschluß an die Regeleinrichtung der Rauchvorrichtung sind die Heizvorrichtungen 8 mit einer entsprechenden Kontaktierung 22 versehen.

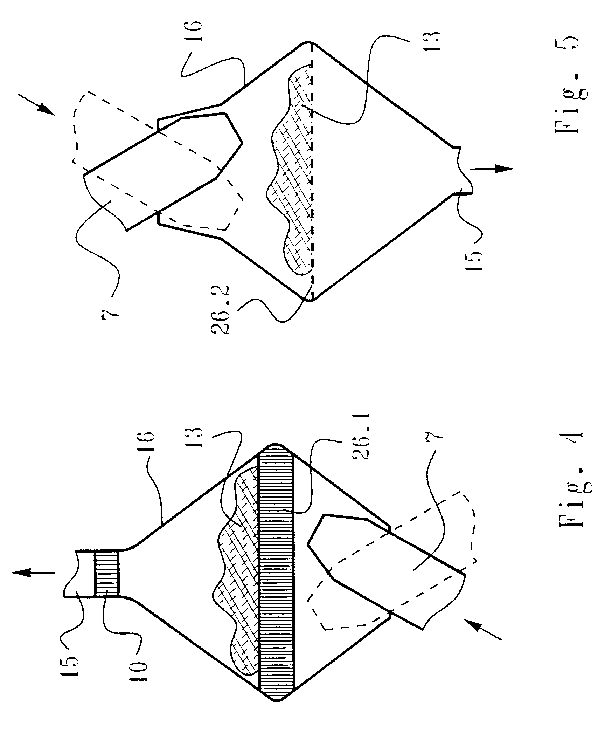

Um die für die optimale Nutzung der Rauchmittelmenge notwendige Relativbewegung zwischen Rauchmittel und Heißluftstrom zu gewährleisten, wird bei nichtbewegtem Rauchmittel eine Bewegung des Heißluftstromes vorgenommen. In den Figuren 4 und 5 sind diese Möglichkeiten als Weiterbildungen der Erfindungen in schematisierter Schnittdarstellung gezeigt. Das Rauchmittel 13 ruht auf einer scheibenförmigen, luftdurchlässig porösen Vorratsfläche 26.1 oder auf einem feinmaschigen Sieb 26.2. Der Heißluftgenerator 7 ist schwenkbar angeordnet, so daß seine Luftaustrittsdüse die luftdurchlässige Auflagefläche 26.1 unterhalb der Rauchmittelmenge 13 (Figur 4) oder die Rauchmittelmenge 13 direkt (Figur 5) flächenmäßig überstreicht.

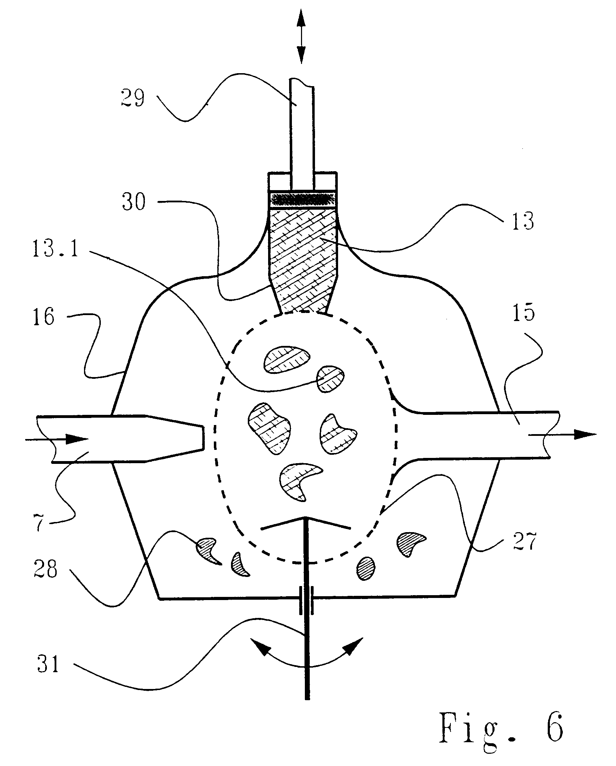

Figur 6 zeigt in schematisierter Darstellung eine vorteilhafte Weiterbildung der Erfindung, wonach die Rauchmittelmenge 13 über einen vorwärmbar ausgebildeten Speicher 30 in einen siebförmigen Vorratsbehälter 27 eingebracht wird und diesen, in Kleinmengen 13.1 aufgeteilt, der Schwerkraft folgend durchläuft. Der siebförmige Vorratsbehälter 27 wird von der aus dem Heißluftgenerator 7 austretenden Heißluft quer zur Bewegungsrichtung der Rauchmittelmengen 13.1 angeströmt. Dadurch wird in einfacher Weise die Relativbewegung zwischen Rauchmittel und Heißluftstrom erreicht und das Rauchmittel optimal zur Aerosolabgabe angeregt. Durch einen Rührknetanker 31 können die Rauchmittelteile innerhalb des Vorratsbehälters 27 im Bedarfsfall noch einmal mit dem Heißlufftstrom in Kontakt gebracht werden, bevor sie endgültig als verbrauchtes Rauchmittel 28 den Behälter 27 verlassen. Durch einen Stempel 29 kann die benötigte Rauchmittelmenge bedarfsweise nachgefördert werden.

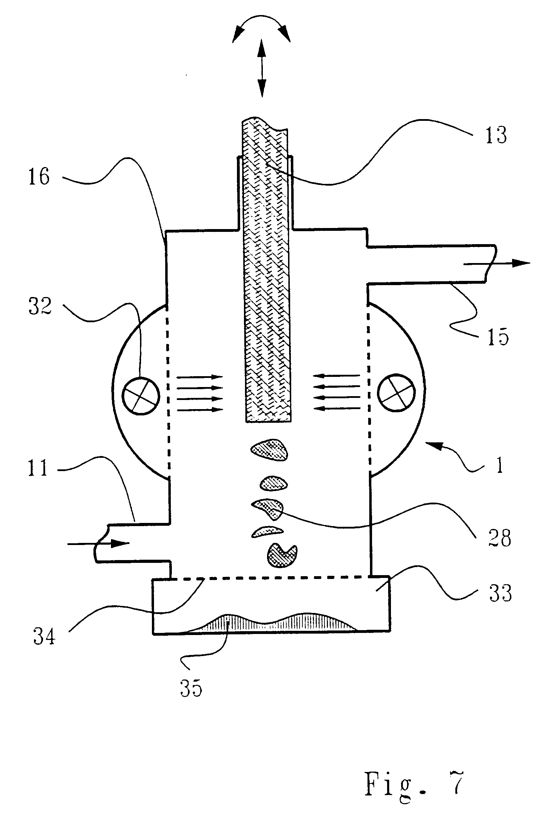

Die in Figur 7 in schematisierter Form dargestellte Ausführungsform der Rauchvorrichtung 1 weist eine Strahlungsheizung 32 auf. Die Strahlung durchdringt das Rauchmittel 13 quer, welches schubweise von oben in die Rauchkammer 16 eingebracht wird. Der Rauchmittelstab 13 "brennt" nach unten ab und gibt dabei die gewünschten Aerosolmengen ab. Die über den Stutzen 11 angesaugte Umgebungsluft durchströmt die Rauchkammer 16 entgegen der Fallrichtung der Aerosole abgebenden und danach verbrauchten Rauchmittelteilmengen 28 und wird am Stutzen 15 mit Aerosolen angereichert durch den Raucher abgesaugt. Die Regelung der Strahlungsintensität erfolgt in gleicher Weise wie die Regelung des Heizstroms in der vorab beschriebenen Weise über die Messung der zum Rauchen benötigten Luftmenge. Zur Reduzierung der Strahlungsleistung ist es günstig, die in die Rauchkammer gesaugte Luft bereits durch einen (nicht dargestellten und vorab beschriebenen) Heißluftgenerator vorzuwärmen. Die verbrauchten Rauchmittelmengen 28 fallen durch ein Rost 34 in einen Sammelbehälter 33 und können als ascheähnlicher Abfall 35 entfernt werden.

Bei einer anderen nicht näher dargestellten Ausführungsvariante der erfindungsgemäßen Vorrichtung ist das Gehäuse im Bereich der Heizvorrichtung derart transparent ausgebildet ist, daß deren Leuchtwirkung bei Erwärmung das Gehäuse durchstrahlt. Die Heizvorrichtung ist dabei vorzugsweise in der Nähe des vom Benutzer entfernten Gehäuseendes angeordnet, so daß der psychologische Eindruck des herkömmlichen Rauchens für den Benutzer hervorgerufen wird. Dieser Effekt kann auch durch eine entsprechende, (üblicherweise rot oder orange) strahlende, Leuchtdiode bewirkt oder unterstützt werden, die zusammen mit der Heizvorrichtung aktiviert wird.

Eine weitere günstige Weiterbildung der Erfindung ermöglicht dem Benutzer sein Rauchprofil individuell wahlweise mehr in Richtung "Zigarette" oder "Wasserpfeife" zu verschieben.

Der entscheidende subjektive Unterschied zwischen diesen klassischen Rauchgeräten besteht für den Raucher vor allem darin, daß bei der Zigarette relativ kleine Mengen hoch konzentriertes Aerosol durch einen Filter oder ein Mundstück zunächst in den Mund- und Rachenraum angesaugt werden, sodann "Frischluft" nachinhaliert wird, die den Rauch in die tiefergelegenen Bronchien- und Luingenbereiche hineintreibt.

Bei der Wasserpfeife hingegen inhaliert der Benutzer größere Mengen Aerosol durch das Gerät und atmet vergleichsweise weniger Frischluft nach.

Im Vergleich zur Wasserpfeife, welche wiederum das andere Extrem darstellt, besitzt die Zigarette bauartbedingt einen relativ hohen Strömungswiderstand, wodurch das typische Gefühl der leicht eingezogenen Backen und Mundwinkel durch den relativ hohen Unterdruck schon bei geringen Volumenströmen entsteht.

Die Erfindung besitzt an sich den konstruktionsbedingten Vorteil eines sehr geringen Strömungswiderstandes, was der Benutzer wie das freie Saugen durch eine größere Wasserpfeife empfindet.

Sofern aber mehr die Kennlinie einer Zigarette gewünscht ist, ermöglicht insbesondere das Aufstecken eines schlauchförmigen, fingerverformbaren Silikonteils auf den Absaugkanal die Angleichung des Rauchprofils durch variables Zusammenpressen während des Rauchens. So lassen sich auf einfache Weise die entscheidenden subjektiv-psychologischen Vorteile der klassischen Rauchgeräte variabel verbinden.

## Patentansprüche

1. Vorrichtung (1) zum Rauchen von Tabak oder eines anderen Rauchmittels (13) oder zum Inhalieren von Aerosolen, welche bei Erwärmung einer aerosolbildenden Substanz gebildet werden, mit
- einem eine im wesentlichen abgeschlossene Kammer (16) bildenden Vorratsbehälter (12; 27) zur Aufnahme des Rauchmittels oder der aerosolbildenden Substanz in zerkleinerter Form,
- einem Generator (7) für erhitztes Trägergas, der eine Heizvorrichtung (8) mit einer Austrittsöffnung (9) zur Einleitung des erhitzten Trägergases in die Kammer aufweist,
wobei das erhitzte Trägergas konvektiv eine Erwärmung des Rauchmittels oder der aerosolbildenden Substanz auf eine Temperatur unterhalb der Gluttemperatur bewirkt und
das Rauchmittel oder die aerosolbildende Substanz in der Kammer derart angeordnet ist und/oder geführt wird, daß die Oberflächen seiner/ihrer Teilchen von dem aus der Austrittsöffnung (9) austretenden Trägergas unmittelbar angeströmt und die Volumina der Teilchen dabei bis zu einer Temperatur in der Nähe, aber unterhalb der Gluttemperatur durchwärmt werden.

2. Vorrichtung (1) zum Rauchen von Tabak oder eines anderen Rauchmittels (13) oder zum Inhalieren von Aerosolen, welche bei Erwärmung einer aerosolbildenden Substanz gebildet werden, mit
- einem eine im wesentlichen abgeschlossene Kammer (16) bildenden Vorratsbehälter zur Aufnahme des Rauchmittels oder der aerosolbildenden Substanz,
- einer unmittelbar auf das Rauchmittel oder die aerosolbildende Substanz einwirkenden Strahlungsheizvorrichtung (32) und
- einer Ansaugöffnung (11) zur Führung eines, wahlweise erhitzten, Trägergases um das Rauchmittel oder die arosolerzeugende Substanz in der Kammer (16) zu einer Absaugöffnung (15) für den Rauch oder das Aerosol,
wobei das Rauchmittel oder die aerosolbildende Substanz derart angeordnet ist und/oder geführt wird, daß es/sie bis zu einer Temperatur in der Nähe, aber unterhalb der Gluttemperatur durchwärmt und in diesem Zustand durch das Trägergas angeströmt wird.

3. Vorrichtung nach Anspruch 1, **dadurch gekennzeichnet**, daß das Rauchmittel (13) auf einer tellerartigen Fläche (12, 26.1, 26.2) in der Kammer (16) in im wesentlichen ebener Erstreckung ausgebreitet ist.

4. Vorrichtung nach Anspruch 3, **dadurch gekennzeichnet**, daß das Rauchmittel durch ein Netz, Sieb oder Gitter (26.2) in seiner Position gehalten ist, wobei die Auflagefläche (26.1) aus einem gesinterten, Filtereigenschaften aufweisenden Material besteht und der Generator (7) derart angeordnet ist, daß das aus der Austrittsöffnung (9) ausströmende Trägergas erst nach Passieren der Auflagefläche (26.1) (26.2) auf das Rauchmittel (13) trifft.

5. Vorrichtung nach einem der vorangehenden Ansprüche, **dadurch gekennzeichnet**, daß der Vorratsbehälter (12) für das Rauchmittel (13) gegenüber dem Trägergasstrom durch Rotation und/oder durch Translation beweglich angeordnet und als einseitig offener Zylinder geringer Höhe oder flacher Quader mit Kammerunterteilung ausgebildet ist.

6. Vorrichtung nach einem der Ansprüche 1 oder 3 bis 5, **dadurch gekennzeichnet**, daß der Generator (7) für erhitztes Trägergas derart angeordnet ist, daß das die Austrittsöffnung (9) verlassende erhitzte Trägergas im wesentlichen die gesamte mit Rauchmittel (13) bedeckte Oberfläche des Vorratsbehälters (12) überstreicht, wobei die Heizvorrichtung (8) insbesondere als Folienheiz-Element (24) ausgebildet ist.

7. Vorrichtung nach einem der Ansprüche 1 oder 3 bis 6, **dadurch gekennzeichnet**, daß das Trägergas an der Austrittsöffnung (9) höchstens kurzzeitig eine Temperatur oberhalb der Gluttemperatur des Rauchmittels (13) aufweist, wobei die Zeitdauer derart bemessen ist, daß unter Berücksichtigung der thermischen Zeitkonstanten der zu erhitzenden Menge des Rauchmittels dessen Gluttemperatur nicht überschritten wird.

8. Vorrichtung nach einem der Ansprüche 1 oder 3 bis 7, **dadurch gekennzeichnet**, daß eine, insbesondere als Programmsteuerung ausgebildete, Regeleinrichtung zur Aufrechterhaltung einer vorgesehenen Austrittstemperatur des Trägergases vorgesehen ist, wobei die Zufuhr von Hilfsenergie in Abhängigkeit von der Menge des die Heizvorrichtung durchströmenden Gases regelbar und/oder für die Messung der durchströmenden Gasmenge ein Drucksensor (6) vorgesehen ist.

9. Vorrichtung nach Anspruch 2, **dadurch gekennzeichnet**, daß ein Transportweg für in Teilmengen (13.1; 28) zerlegtes Rauchmittel (13) vorgesehen ist, bei dem die Teilmengen der Schwerkraft folgend den Trägergasstrom im wesentlichen senkrecht zu dessen Strömungsrichtung oder im Gegenstrom durchqueren.

10. Vorrichtung nach einem der vorangehenden Ansprüche, **dadurch gekennzeichnet**, daß deren Gehäuse im Bereich der Heizvorrichtung (8, 32) transparent ausgebildet ist, so daß deren Leuchtwirkung bei Erwärmung das Gehäuse durchstrahlt und/oder daß mindestens ein elektrisches Leuchtmittel vorgesehen ist, welches zusammen mit der Heizvorrichtung aktiviert wird.

11. Vorrichtung nach einem der vorangehenden Ansprüche, **dadurch gekennzeichnet**, daß im raucherseitigen Absaugkanal mindestens ein variabel querschnittsverengbarer Bereich vorgesehen ist, insbesondere als fingerverformbares Elastomerteil.

## Claims

1. A device (1) for smoking tobacco or another smoking product (13) or for the inhalation of aerosols released by the heating of an aerosol releasing substance, comprising
- a reservoir (12; 27) forming a substantially closed chamber (16) for containing the smoking product or the aerosol releasing substance in crushed or finely distributed form, and
- a hot carrier gas generator (7) comprising a heating appliance (8) with an outlet (9) for introducing the heated carrier gas into the chamber,
wherein the heated carrier gas causes a heating by convection of the smoking product or the aerosol releasing substance to a temperature below that of the product's glow temperature and
the smoking product or the aerosol releasing substance is arranged in the chamber and/or guided in such a way that the surfaces of its particles will be directly affected by the carrier gas stream emitted from the outlet (9), and the volumes of the particles will be warmed through up to a temperature close to but lower than said glow temperature.

2. A device (1) for smoking tobacco or another smoking product (13) or for the inhalation of aerosols released by the heating of an aerosol releasing substance, comprising
- a reservoir forming a substantially closed chamber (16) for containing the smoking product or the aerosol releasing substance,
- a radiation heating appliance (32) directly affecting the smoking product or the aerosol releasing substance, and
- a suck-in opening (11) for guiding a carrier gas being optionally heated around the smoking product or the aerosol releasing substance within the chamber (16) to a suck-off opening (15) for the smoke or the aerosol,
wherein the smoking product or the aerosol generating substance is arranged and/or guided in such a way that it will be warmed through up to a temperature close to but lower than said glow temperature and will be streamed along by the carrier gas in this state.

3. A device according to claim 1 wherein the smoking product (13) is distributed on a saucer-like surface (12; 26.1, 26.2) within the chamber (16) in a substantially even and flat manner.

4. A device according to claim 3 wherein the smoking product is held in position by a mesh, sieve or grille (26.2), wherein the supporting surface (26.1) consists of a sintered material with the ability to act as a filter and/or where the carrier gas generator (7) is mounted in such a way that the carrier gas flowing from the outlet (9) subsequently to having passed through the supporting surface (26.1, 26.2) strikes the smoking product (13).

5. A device according to one of the previous claims wherein the reservoir (12) is, through rotation and/or translation, moveable and formed as a shallow, one-sided open cylinder or a flat cuboid with compartments (14).

6. A device according to one of the claims 1 or 3 to 5 wherein the hot carrier gas generator (7) is mounted so that the heated carrier gas leaving the outlet (9) is roughly able to strike the entire surface area of the reservoir (12) covered with the smoking product (13), wherein the heating appliance (8) preferably is a heating foil element (24).

7. A device according to one of the claims 1 or 3 to 6 wherein the carrier gas at the outlet (9) at the most for a short time achieves a temperature above that of the smoking product's (13) glow temperature, whereby the period of time is measured in such a way that the glow temperature of the smoking product to be heated will not be exceeded, taking into account the thermal time constants.

8. A device according to one of the claims 1 or 3 to 7 wherein, especially programmable, control means for maintaining a predetermined outflow temperature of the carrier gas is provided, wherein the supply of auxiliary energy is adjustable dependant upon the quantity of gas flow through the heating appliance and/or in which a pressure sensor (6) is provided to measure the gas flow rate.

9. A device according to claim 2 in which a passage for the sub-quantities (13.1; 28) of the smoking product (13) is provided through which the gravitationally descending sub-quantities pass and cross the carrier gas stream roughly perpendicular or antiparallel to its flow direction.

10. A device according to one of the previous claims in which the housing adjacent to the heating appliance is transparent so that the heat glow is visible through the housing and/or that at least one electrical light is provided which is activated with the heating appliance.

11. A device according to one of the previous claims wherein within the suck-off channel on the smoker's side at least one portion of contractable cross-section is provided, especially being arranged as an elastomer part being deformable by finger pressure.

## Revendications

1. Dispositif (1) pour fumer du tabac ou un autre produit à fumer (13) ou bien pour inhaler des aérosols qui sont formés lors du réchauffement d'une substance formant des aérosols, comportant
- un récipient de réserve (12 ; 27) formant une chambre (16) sensiblement refermée, pour recevoir le produit à fumer ou la substance formant de l'aérosol sous forme fragmentée,
- un générateur (7) pour un gaz porteur réchauffé, qui comprend un dispositif de chauffage (8) présentant une ouverture de sortie (9) pour introduire le gaz porteur réchauffé dans la chambre,
dans lequel le gaz porteur réchauffé assure un réchauffement par convection du produit à fumer ou de la substance formant des aérosols à une température inférieure à la température d'incandescence, et
dans lequel le produit à fumer ou la substance formant des aérosols est agencé(e) et/ou guidé(e) de telle sorte dans la chambre que les surfaces de ses particules sont directement attaquées par le gaz porteur sortant hors de l'ouverture de sortie (9) et que les volumes des particules sont réchauffés de part en part jusqu'à une température proche de, mais inférieure à, la température d'incandescence.

2. Dispositif (1) pour fumer du tabac ou un autre produit à fumer (13) ou bien pour inhaler des aérosols qui sont formés lors du réchauffement d'une substance formant des aérosols, comportant
- un récipient de réserve formant une chambre (16) sensiblement refermée pour recevoir le produit à fumer ou la substance formant des aérosols,
- un dispositif de chauffage à rayonnement (32) agissant directement sur le produit à fumer ou la substance formant des aérosols, et
- une ouverture d'aspiration (11) pour guider un gaz porteur éventuellement réchauffé autour du produit à fumer ou la substance formant des aérosols dans la chambre (16) jusqu'à une ouverture d'aspiration (15) pour la fumée ou pour l'aérosol,
dans lequel le produit à fumer ou la substance formant des aérosols est agencé(e) et/ou guidé(e) de telle sorte qu'il/qu'elle est réchauffé(e) de part en part jusqu'à une température proche de, mais inférieure à, la température de braise et qu'il/qu'elle est attaqué(e) dans cet état par le gaz porteur.

3. Dispositif selon la revendication 1, caractérisé en ce que le produit à fumer (13) est répandu sur une surface en forme de disque (12, 26.1, 26.2) dans la chambre (16) sensiblement en extension à plat.

4. Dispositif selon la revendication 3, caractérisé en ce que le produit à fumer est retenu dans sa position par un filet, un tamis ou une grille (26.2), la surface d'appui (26.1) étant constituée en un matériau fritté présentant des propriétés de filtrage, et en ce que le générateur (7) est agencé de telle sorte que le gaz porteur sortant hors de l'ouverture de sortie (9) tombe sur le produit à fumer (13) uniquement après avoir passé la surface d'appui (26.1, 26.2).

5. Dispositif selon l'une quelconque des revendications précédentes, caractérisé en ce que le récipient de réserve (12) pour le produit à fumer (13) est agencé de façon mobile en rotation et/ou en translation par rapport au courant de gaz porteur, et en ce qu'il est réalisé sous forme d'un cylindre ouvert sur un côté et de faible hauteur ou sous forme d'un parallélépipède présentant une subdivision en chambres.

6. Dispositif selon l'une quelconque des revendications 1 ou 3 à 5, caractérisé en ce que le générateur (7) pour le gaz porteur réchauffé est agencé de telle sorte que le gaz porteur réchauffé quittant l'ouverture de sortie (9) balaie sensiblement toute la surface, recouverte de produit à fumer (13), du récipient de réserve (12), le dispositif de chauffage (8) étant réalisé en particulier sous forme d'un élément chauffant en film (24).

7. Dispositif selon l'une quelconque des revendications 1 ou 3 à 6, caractérisé en ce que le gaz porteur présente au niveau de l'ouverture de sortie (9) tout au plus brièvement une température supérieure à la température d'incandescence du produit à fumer (13), la durée temporelle étant choisie de telle sorte que, en tenant compte de la constante temporelle thermique de la quantité à réchauffer du produit à fumer, sa température d'incandescence n'est pas dépassée.

8. Dispositif selon l'une quelconque des revendications 1 ou 3 à 7, caractérisé en ce qu'il est prévu un dispositif de régulation, réalisé en particulier sous forme d'une commande par programme, pour maintenir une température de sortie prévue du gaz porteur, l'amenée d'une énergie auxiliaire étant réglable en fonction de la quantité du gaz traversant le dispositif de chauffage et/ou en ce qu'il est prévu un détecteur de pression (6) pour mesurer la quantité de gaz en écoulement traversant.

9. Dispositif selon la revendication 2, caractérisé en ce qu'il est prévu un trajet de transport pour le produit à fumer (13) fragmenté en quantités partielles, dans lequel les quantités partielles traversent, en suivant la pesanteur, le courant de gaz porteur sensiblement perpendiculairement à sa direction d'écoulement, ou bien à contre-courant.

10. Dispositif selon l'une quelconque des revendications précédentes, caractérisé en ce que son boîtier est réalisé transparent dans la région du dispositif de chauffage (8, 32), de sorte que son effet luminescent traverse le boîtier lors d'un réchauffement et/ou en ce qu'il est prévu au moins un organe lumineux électrique qui est activé conjointement avec le dispositif de chauffage.

11. Dispositif selon l'une quelconque des revendications précédentes, caractérisé en ce qu'il est prévu dans le canal d'aspiration côté fumeur au moins une région dont la section transversale est rétrécissable de façon variable, en particulier sous forme d'une pièce élastomère déformable à l'aide des doigts.
